Europäisches Patentamt

European Patent Office

Office européen des brevets

(19)

(11) Publication number: **0 256 099 B1**

# EUROPEAN PATENT SPECIFICATION

(12)

(45) Date of publication of patent specification: **15.04.92**   (51) Int. Cl.⁵: **H03G 3/20**

(21) Application number: **87901402.5**

(22) Date of filing: **03.02.87**

(86) International application number:
**PCT/DK87/00010**

(87) International publication number:
**WO 87/04877 (13.08.87 87/18)**

(54) **A METHOD FOR AUTOMATIC GAIN CONTROL OF A SIGNAL.**

(30) Priority: **11.02.86 DK 651/86**

(43) Date of publication of application:
**24.02.88 Bulletin 88/08**

(45) Publication of the grant of the patent:
**15.04.92 Bulletin 92/16**

(84) Designated Contracting States:
**BE FR IT**

(56) References cited:
**EP-A- 0 153 719**
**GB-A- 2 048 621**

(73) Proprietor: **JORGENSEN, Poul Richter**
**Saugskaervej 16 Thuroe**
**DK-5700 Svendborg(DK)**

(72) Inventor: **JORGENSEN, Poul Richter**
**Saugskaervej 16 Thuroe**
**DK-5700 Svendborg(DK)**

(74) Representative: **Nony, Michel et al**
**Cabinet Nony 29, rue Cambacérès**
**F-75008 Paris(FR)**

Rank Xerox (UK) Business Services

EP 0 256 099 B1

## Description

The invention relates to a method for automatic gain control ('AGC') of a signal, the method being of the kind specified in the preamble of claim 1.

In the event of gain control of Prior Art the gain control is performed depending on e.g. mean value, effective value or peak value of the input signal. The Prior Art gain control can be implemented by circuits consisting exclusively of analog circuit devices, by circuits containing both analog circuit devices and digital circuit devices, or by circuits containing only digital circuit devices. AGC on an analog basis or on an analog/digital basis is a rather slow process due to the implemented circuits (feed-back circuits) but irrespective of the type of circuit, it contains circuit devices which detect a single characteristic parameter of the input signal to be adjusted, e.g. as mentioned the mean value, effective value or peak value of the signal.

GB-A- 2 048 621 includes a circuit for automatic gain control for processing audio signals as input signals to the system together with noise signals for provision of the maximum gain.

By the circuit only a rather slow sampling and hold process is provided, and the gain control takes place before an A/D conversion, for which reason the reproduction of the waveform is inaccurate. Further, the circuit is only constituted for suppression of not too fast noise signals (audio signals), and is unable to suppress electrical noise effectively.

According to EP-A-0 153 719 the amplification in an AGC circuit is provided in a process with two steps, by a pre-amplifier and by the output signal from a computer. The circuit shall be able to maintain a constant total gain and will constantly control and change the gain ratio of the two amplifiers.

Thus, a constant total gain is provided with a limited amount of different gain levels. As a matter of experience, a cyclic variation of the gain will occur in such a circuit when processing signals at a level close to the maximum.

Further, the circuit has no noise suppression.

With applications where the further processing of the gain-controlled signal is in digital mode, e.g. in a microprocessor system, it is a problem to process an analog signal having a large dynamic range by a predetermined digital word length.

In certain situations this signal may, furthermore, have an undesired signal superimposed, which undesired signal may originate e.g. from interference sources, and the amplitude of which is higher than the signal required. This undesired signal will, therefore, be able to reduce the sensitivity of the automatic gain control, and so it will be expedient to remove the undesired signal before the automatic gain control is performed.

It is, therefore, the objective of the invention in connection with a desired analog signal converted into digital mode and with a frequency within a prefixed narrow frequency range and a varying signal level, to provide an increased dynamic range with a prefixed digital word length.

The objective specified is achieved by a method of the kind dealt with in the introduction which according to the invention is characteristic by the measures specified in the characterizing part of claim 1. A special embodiment of the method is specified in claim 2.

When the signal level of the signal exceeds its upper limit value $G_2$ during its increase, the signal is attenuated in increments until its highest instantaneous level is below $G_2$. If the signal level of the signal when decreasing comes below the lower limit value $G_1$, the signal is amplified (also in increments) until its highest instantaneous level exceeds $G_1$. Hence it is achieved that the dynamic range of the gain-controlled signal can always be adapted to a prefixed digital word length so that it does not cause overload or in some other way creates irregularities in operation in the subsequent processing circuit.

Through the specified design of the automatic gain-controlled circuit the microprocessor can, therefore, adjust the gain of the analog input signal depending on a prefixed expectation of the temporal sequence of the input signal, a so-called fixed intelligence. Furthermore, the microprocessor can adjust the gain depending on its own experience of the temporal sequence of the input signal, a so-called adaptive intelligence.

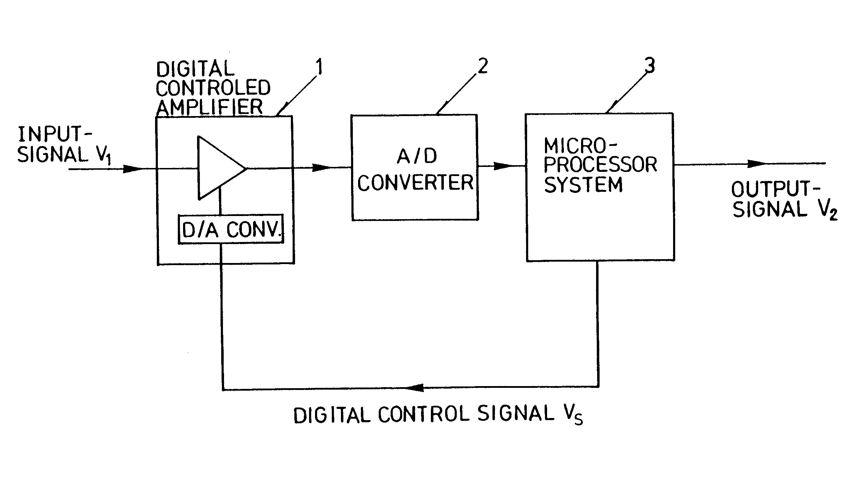

In what follows, the invention is explained in greater detail with reference to the drawing showing a circuit for automatic gain control for implementation of the method according to the invention. As shown on the drawing, an analog signal $v_1$ is supplied to an analog amplifier 1 whose gain factor can be adjusted in increments by means of a digital control signal $v_s$ which is supplied to the gain control input of the analog signal amplifier 1. An amplified analog signal is emitted by the output of the analog amplifier 1 to the input of an analog-to-digital converter 2. The analog-to-digital converter 2 samples the amplified analog signal and produces, at its output, a digital signal representation of the input signal $v_1$ comprising sampled instantaneous levels of the amplified analog signal at successive sampling times.

The analog input signal $v_1$ is a signal with a varying signal level which after the amplification in the automatic gain control circuit has to be within prefixed upper and lower limits respectively which with reference to the signal input are called $G_2$ and $G_1$ respectively. In a high number of cases the signal lies within a prefixed narrow frequency interval, but the invention need not be limited to a narrow frequency interval.

The digital signal representation is supplied to the data input on a Prior Art microprocessor device 3, and the digital signal representation is stored in storage locations in a storage device in the microprocessor 3. The sampling and storage of the signal take place in a socalled measurement period of a duration corresponding to a given number of signal periods. A measurment period is followed by an adjustment period of a prefixed duration in which the signal is gain-controlled if necessary, but without sampling and storage of the signal taking place during this period. The adjustment period is followed by a measurement period in which the signal is sampled and stored again, and this measurement period is again followed by an adjustment period, etc., so that a measurement period and an adjustment period alternate. This division may, for example, be made by a synchronization signal, but can also be carried out in other suitable ways. Continuous adjustment of the signal amplitude is thus not implemented as in the case of Prior Art automatic gain control with analog circuit devices where the gain control is implemented depending on e.g. mean value, effective value or peak value of the input signal, but the adjustment is limited to prefixed periods, viz. the said adjustment periods.

During the measurement period the microprocessor device 3 processes the digital signal representation stored in the storage device in accordance with the stored program and supplies, during a subsequent adjustment period which need not be the adjustment period following immediately after the measurement period, but may be an arbitrary adjustment period following a measurement period, a digital control signal $v_s$ to the gain control input of the digitally controlled amplifier in accordance with this program. The digital control signal $v_s$ controls the gain of the amplifier 1 in increments.

The processing performed by the microprocessor device 3 of the received and stored signal may consist of a comparison of the digital signal representation stored in the storage device with an upper limit value $G_2$ and lower limit value $G_1$ respectively coded into the microprocessor device 3, both these limit values being referred to the signal input.

If the microprocessor device 3 ascertains during the measurement period that the signal level of the signal is increasing, the instantaneous levels of the stored digital signal representation are compared to the upper limit value $G_2$, and if the highest value of the instantaneous levels exceeds the upper limit value $G_2$, the microprocessor device 3 emits a digital control signal $v_s$ to the gain control input of the digitally controlled amplifier 1, of such a value that the gain is adjusted (in increments) in a direction which attenuates the signal $v_2$. This stepwise adjustment of the gain of the amplifier 1 continues until the highest instantaneous level of the signal is lower than $G_2$. If during the measurement period the microprocessor device 3 ascertains that the signal level of the signal is decreasing, the instantaneous levels of the stored digital signal representation are compared to the lower limit value $G_1$, and if the highest value of the instantaneous levels comes below the lower limit value $G_1$, the microprocessor device 3 emits a digital control signal $v_s$ to the gain control input of the digitally controlled amplifier 1 of such a value that the gain is adjusted in increments in a direction which amplifies the signal $v_2$. This stepwise adjustment of the gain of the amplifier 1 continues until the upper instantaneous level of the signal is above $G_1$. That is, an adjustment of the signal is implemented either if the highest instantaneous level of an increasing signal level exceeds the upper limit value $G_2$, or if the highest instantaneous level of a decreasing signal level comes below the lower limit value $G_1$. For example, if the highest instantaneous level of a decreasing signal level comes below the upper limit value $G_2$, no adjustment takes place. By means of the method described in the foregoing the result is that the highest instantaneous level of the adjusted signal (output signal $v_2$) will always be between the lower limit value $G_1$ and the upper limit value $G_2$ so that the dynamic range of the signal can always be adapted to a prefixed digital word length so that it does not cause overload or create any other irregular operation in any subsequent processing circuit. This mode of adjustment corresponds to the above mentioned socalled 'fixed intelligence'. The adjusted output signal $v_2$ can also be used internally for other purposes, e.g. as a control signal. As the gain of the digitally controlled amplifier 1 is adjusted in increments during the adjustment periods, any distortion of the output signal $v_2$ in connection with the adjustment is limited to these periods which are already known.

The processing performed by the microprocessor device 3 of the signal received and stored may also consist of a computation e.g. of the frequency of the input signal $v_1$ and a subsequent adjustment on the basis of this frequency value as explained in the following. If e.g. an undesired signal (an interference signal) occurs which is superimposed on the input signal required, and whose amplitude is higher than the input signal required, this undesired signal will reduce the sensitivity of the automatic gain control because the amplitude of the undesired signal is higher than that of the desired input signal, and the adjustment, therefore, takes place depending on the higher amplitude of this undesired signal. This can be avoided by the microprocessor device 3 analysing, during the measurement period, the digital signal representation of the total signal - i.e. both the undesired signal and the desired signal - stored in the storage locations of the storage device, and on the basis of its knowledge of the input signal frequency obtained in the above-mentioned way, i.e. based on its own experience of the development of the input signal, determines the frequency of the undesired signal and, if this frequency differs from that of the desired input signal, then deletes the storage locations of the undesired signal in the storage device, whereupon the adjustment of the input signal will take place in the abovementioned way. Hence it is avoided that the automatic gain control sensitivity is reduced as a result of a large interference signal. This mode of adjustment corresponds to the above-mentioned "adaptive intelligence".

This mode of adjustment can be developed so that the adjustment takes place dependent e.g. on both amplitude, frequency and phase, i.e. depending on arbitrarily complicated functions of the input signal.

Even if it not shown on the drawing, the synchronisation signals required for operation of the circuit as well as power and voltage levels are supplied to each individual circuit device of the automatic gain-controlled circuit.

## Claims

1. A method for automatic gain control of an amplifier receiving an analog input signal comprising the steps of during a measurement period producing a digital signal corresponding to the input signal, producing a control signal in response to the desired signal and varying the gain of the amplifier in response to said control signal, and storing the digital signal representation, characterized in that each individual sampled instantaneous level of the digital signal belonging to the successive sampling times is compared during the measurement period to an upper limit value ($G_2$) when the input signal has an increasing signal level and to a lower limit value ($G_1$) when the input signal has a decreasing signal level, and if the highest instantaneous levels of the digital signal exceed the upper limit value or come below the lower limit value, the said control signal is produced in an adjustment period following the measurement period at such a value that the signal level is situated below the upper limit value and above the lower limit value.

2. A method for automatic gain control as claimed in claim 1 and where the analog signal includes a first analog signal which is to be gain-controlled, and a second undesired analog signal whose amplitude is higher than that of the first analog signal, characterized by computation of the frequency of the first analog signal on the basis of the previously stored values of the digital signal representation of the first analog signal, determination of the frequency of the second analog signal on the basis of the stored values of the total digital signal representation of the first and second analog signals, as well as deletion of the stored values of the second analog signal if the frequency of the second analog signal differs from the frequency of the first analog signal, whereupon the automatic gain control takes place as specified in the characterising portion of claim 1.

## Revendications

1. Procédé de commande automatique de gain d'un amplificateur recevant un signal d'entrée analogique, comportant les étapes consistant à, durant une période de mesure, produire un signal numérique correspondant au signal d'entrée, produire un signal de commande en réponse au signal désiré et modifier le gain de l'amplificateur en réponse audit signal de commande, et mémoriser la représentation du signal numérique, caractérisé en ce que chaque niveau instantané échantillonné individuel du signal numérique appartenant à des instants d'échantillonnage successifs est comparé durant la période de mesure à une valeur limite supérieure ($G_2$) lorsque le signal d'entrée possède un niveau de signal croissant et à une valeur limite inférieure ($G_1$) lorsque le signal d'entrée possède un niveau de signal décroissant, et si les niveaux instantanés les plus élevés du signal numérique excèdent la valeur limite supérieure ou passent au-dessous de la valeur limite inférieure, ledit signal de commande est produit au cours d'une période de réglage suivant la période de mesure à une valeur telle que le niveau de signal soit situé au-dessous de la valeur limite supérieure et au-dessus de la valeur limite inférieure.

2. Procédé de commande automatique de gain selon la revendication 1 et où le signal analogique comprend un premier signal analogique dont le gain doit être commandé, et un second signal analogique indésirable dont l'amplitude est supérieure à celle du premier signal analogique, caractérisé par le calcul de la fréquence du premier signal analogique en fonction des valeurs préalablement mémorisées de la représentation du signal numérique du premier signal analogique, la détermination de la fréquence du second signal analogique en fonction des valeurs mémorisées de la représentation de signal numérique total du premier et second signaux analogiques, ainsi que la suppression des valeurs mémorisées du second signal analogique si la fréquence du second signal analogique diffère de la fréquence du premier signal analogique, après quoi la commande automatique de gain est effectuée comme énoncé dans la partie caractérisante de la revendication 1.

**Patentansprüche**

1. Verfahren zur automatischen Verstärkungssteuerung eines ein analoges Eingangssignal empfangenden Verstärkers, das die Schritte umfasst, während eines Messungszeitabschnittes ein digitales, dem Eingangssignal entsprechendes Signal zu erzeugen, in Erwiderung auf das gewünschte Signal ein Steuersignal zu erzeugen und die Verstärkung des Verstärkers in Erwiderung auf das genannte Steuersignal zu verändern, und die digitale Signaldarstellung zu speichern, **dadurch gekennzeichnet**, dass jeder einzelne abgetastete momentane Pegel des zu den aufeinanderfolgenden Abtastzeiten gehörenden Digitalsignals während des Messungszeitabschnittes verglichen wird mit einem oberen Grenzwert ($G_2$), wenn das Eingangssignal einen steigenden Signalpegel aufweist, und mit einem unteren Grenzwert ($G_1$), wenn das Eingangssignal einen fallenden Signalpegel aufweist, und wenn die grössten momentanen Pegel des Digitalsignals einen oberen Grenzwert überschreiten oder unter den unteren Grenzwert fallen, und dass das genannte Steuersignal in einem Einstellungszeitabschnitt erzeugt wird, der dem Messungszeitabschnitt bei einem solchen Wert folgt, dass der Signalpegel unter dem oberen Grenzwert und über dem unteren Grenzwert liegt.

2. Verfahren zur automatischen Verstärkungssteuerung gemäss Anspruch 1, bei dem das elektrische analoge Signal einschliesst ein erstes elektrisches analoges Signal, das verstärkungsgesteuert werden soll, und ein zweites unerwünschtes elektrisches analoges Signal, dessen Amplitude grösser ist als die des ersten analogen Signals, **dadurch gekennzeichnet**, dass die Frequenz des ersten analogen Signals auf der Basis der zuvor gespeicherten Werte der digitalen Signaldarstellung des ersten analogen Signals berechnet wird, dass die Frequenz des zweiten analogen Signals auf der Basis der gespeicherten Werte der gesamten digitalen Signaldarstellung des ersten und zweiten analogen Signals bestimmt wird, und dass die gespeicherten Werte des zweiten analogen Signals gelöscht werden, wenn sich die Frequenz des zweiten analogen Signals von der Frequenz des ersten analogen Signals unterscheidet, wonach die automatische Verstärkungssteuerung wie in Anspruch 1 (Kennzeichen) angegeben erfolgt.

EP 0 256 099 B1

DIGITAL
CONTROLED
AMPLIFIER 1

2

3

INPUT-
SIGNAL $V_1$

A/D
CONVERTER

MICRO-
PROCESSOR
SYSTEM

D/A CONV.

OUTPUT-
SIGNAL $V_2$

DIGITAL CONTROL SIGNAL $V_S$